# EUROPEAN PATENT APPLICATION

(11) **EP 4 470 550 A1**
(43) Date of publication of application: **04.12.2024**
(21) Application number: 22923357.2
(22) Date of filing: 27.10.2022
(51) Int. Cl.: A61K 38/00, A61K 47/00, A61K 9/00

(54) **PROTEIN FORMULATION, PREPARATION METHOD THEREFOR AND USE THEREOF**

(30) Priority: 27.01.2022 CN 202210101468
(71) Applicant: Beijing Liangyuan Bio-Science, LLC, Beijing 100085 (CN)
(72) Inventor: TANG, Ning, Beijing 100085 (CN); WEI, Xiuli, Beijing 100085 (CN)
(74) Representative: J A Kemp LLP
(86) International application number: PCT/CN2022/128021
(87) International publication number: WO 2023/142559

(57) **Abstract**

The present invention relates to a protein formulation, a preparation method therefor and a use thereof. The formulation is an aqueous solvent, and comprises: (1) a cytokine protein having a concentration of greater than 1 mg/ml; (2) polyethylene glycol)-distearoylphosphatidylethanolamine (PEG-DSPE); and (3) an essential pH regulator/buffer. The cytokine includes but is not limited to: IL2, IL4, IL7, IL9, IL10, IL15, IL21, G-CSF, GM-CSF, IFN, EPO, GF, and TNF. The formulation is used for preparing drugs or formulations administered by means of subcutaneous injection.

## Description

### CROSS REFERENCE TO RELATED APPLICATIONS

The present application claims benefit of priority to Chinese patent application No. 202210101468.4, filed to the CNIPA on January 27, 2022, which are incorporated herein by reference in its entirety.

### FIELD OF THE INVENTION

The invention belongs to the field of biotechnology, and specifically relates to a protein formulation, preparation method and use thereof.

### BACKGROUND OF THE INVENTION

Cytokines (CKs) are small molecular polypeptides or glycoproteins synthesized and secreted by various tissue cells (mainly immune cells). Cytokines can mediate the interaction between cells and have a variety of biological functions, such as regulation of cell growth, differentiation and maturation, function maintenance, regulation of immune response, participation of inflammatory response, wound healing and regression of tumor growth, etc. Since the discovery of interferon by Lssac in 1957, more than 200 cytokines have been discovered so far. All interleukins, interferons, tumor necrosis factors, hematopoietic factors, growth factors, chemokines, etc. are collectively referred to as cytokines.

### 1. According to different functions, cytokines are broadly classified as follows:

(1) Interleukins (ILs) are cytokines produced by lymphocytes, monocytes or other non-mononuclear cells, and play an important role in the regulation of intercellular interactions, immunomodulation, hematopoiesis and inflammation.
(2) Colony stimulating factors (CSFs) are named as G (granulocyte)-CSF, M (macrophage)-CSF, GM (granulocyte and macrophage)-CSF, etc., respectively, according to their roles in stimulating hematopoietic stem cells or hematopoietic cells at different differentiation stages. Different CSFs can not only stimulate the proliferation and differentiation of hematopoietic stem cells and progenitor cells at different developmental stages, but also promote the function of mature cells.
(3) Interferons (IFNs) can be divided into IFN-α, IFN-β and IFN-γ according to their source and structure, and have antiviral, antitumor and immunomodulatory effects.
(4) Tumor necrosis factors (TNFs) can be divided into TNF-α and TNF-β. The former is produced by mononuclear-macrophages, and the latter is produced by activated T cells. The two types of TNF have similar basic biological activities, including immunomodulation, involvement in the occurrence of fever and inflammation, in addition to tumor cell killing.
(5) Growth factors (GFs), such as epidermal growth factor (EGF), platelet-derived growth factor (PDGF), fibroblast growth factor (FGF), hepatocyte growth factor (HGF), insulin-like growth factor-I (IGF-1), IGF-II, leukemia inhibitory factor (LIF), nerve growth factor (NGF), oncostatin M (OSM), platelet-derived endothelial cell growth factor (PDECGF), transforming growth factor-α (TGF-α), vascular endothelial growth factor (VEGF), etc.
(6) Others, such as transforming growth factor-β family (TGF-β family), chemokine family, erythropoietin (EPO), etc., also have different biological functions.

### 2. Common structure of cytokines

In terms of molecular structure, **cytokines are small polypeptides, most of which are composed of about 100 amino acids.** By specifically binding to cytokine receptors on the surface of target cells, cytokines can exert their biological effects, which include promotion of the proliferation and differentiation of target cells, enhancement of anti-infection and tumor cell killing effects, promotion or inhibition of the synthesis of other cytokines, promotion of inflammatory processes, and influence of cell metabolism, etc. These effects of cytokines have characteristics of network, that is, each cytokine can act on a variety of cells, each cell can be regulated by multiple cytokines, and different cytokines have synergistic or restrictive effects on each other, which constitute a complex cytokine immunomodulation network. Understanding of this network is far from clear.

### 3. Cytokine pharmaceuticals and their development difficulties

Interferon α, which was first used in clinic, has achieved significant efficacy in the treatment of leukemia and viral infections. In China, interferon α passed the review of new drugs in 1991 and has been widely used.

The internationally approved cytokine drugs also include EPO, interferon γ, GM-CSF, G-CSF, IL-2, etc.

Obviously, compared with a large number of naturally occurring cytokines, the development of cytokine pharmaceuticals is very slow, and the process of preparing cytokine pharmaceuticals is extremely difficult. Specifically, the difficulties in the development of cytokine pharmaceuticals mainly include the following:
(1) Cytokine proteins are unstable in structure and prone to aggregate, resulting in poor water solubility and poor stability of the proteins;
(2) Cytokine proteins usually have a short half-life and poor stability, and are easily degraded *in vivo,* which makes it difficult to control the therapeutic window of cytokine pharmaceuticals;
(3) Cytokine proteins are often able to activate a variety of different receptors or receptor combinations, resulting in non-specific pharmacological effects and relatively strong side effects;

### Therefore, the development of cytokine pharmaceuticals requires a new idea and formulation system.

### 4. Interleukin 2 (IL2)

Interleukin 2 (IL2), also known as T cell growth factor, was discovered in 1976 and stimulates T cell clonal expansion in *in vitro* experiments. IL2 is a glycoprotein comprised of four α-helices with a molecular weight of 15.5 Kd, which is mainly secreted by activated CD4 T cells, while other cells such as activated CD8 cells, NK cells, NKT cells and ILCs can also produce a small amount of IL2 [3, 4]. IL2 functions by binding to its receptor cells through autocrine and paracrine pathways, and plays an important role in regulating T cells and NK cells. The receptor for IL-2 is composed of three subunits: IL2Rα (CD25), IL2Rβ (CD122) and IL2Ryc (also known as the common cytokine receptor γ chain, yc or CD 132), and the affinity between IL2 and the three subunits is different. IL2 binds to the α subunit with low affinity (Kd~10⁻⁸ M), binds to the βγ dimer with medium affinity (Kd~10⁻⁹ M), and binds to the αβγ trimer with high affinity (Kd~10⁻¹¹ M). In addition, IL2Ryc (CD132) is also the receptor unit for IL4, IL7, IL9, IL15 and IL21.

The expression levels of receptors with different affinities are different on different cells: βγ receptors are mainly expressed on resting T cells, CD8 T memory cells and NK cells; α receptors are up-regulated after cell activation, and activated T cells express αβγ receptors with high-affinity. Tregs consistently express α receptors at high levels, and have a high affinity for IL2. Due to differences in receptor expression, low-dose IL2 preferentially activates Tregs expressing high-affinity receptors, and only high-dose IL2 can effectively activate CD8+ T cells and NK cells. However, the clinical use of high-dose IL2 will lead to greater toxic side effects, which limits the use of IL2.

In order to overcome this problem, IL2 molecules have been modified by different means to reduce their binding to Tregs and increase their binding to effector T cells and NK cells, so as to enhance their efficacy in tumor treatment and avoid the toxicity caused by a high dose. In the prior art, the IL2 modification methods mainly include:
(1) PEGylated IL2
   IL2 has a molecular weight of 15.5 KD and a half-life of 10-85 min in serum *in vivo.* Multiple repeated administrations of IL2 are required to achieve the therapeutic effect. PEGylated IL2 increases the overall molecular weight of IL2 and prolongs the half-life of IL2. At the same time, the difference in the modification site of PEGylation can make IL2 prefer to bind IL2-Rα or IL2-Rb.
(2) Complex of IL-2 and anti-IL-2 antibody
   On the one hand, the combination of IL2 and anti-IL2 antibody can prolong the half-life of IL2. On the other hand, because anti-IL2 antibody can bind to different sites of IL2 molecules, the complex of IL2 and anti-IL2 antibody shows different binding preferences to receptors CD25 or CD122.
(3) IL2 mutation modification
   The affinity of IL2 to different receptor subunits can also be changed by point mutation modification of different receptor binding sites on IL-2. By point mutation of five amino acids of IL-2, namely L80F, R81D, L85V, I86V and I92F, the conformation of IL-2 is changed, which increases the binding of IL-2 to CD122 and make the downstream signal transduction independent of CD25. Mutated IL2 tends to expand CD8+ T cells and NK cells, has lower toxic and side effects and shows better tumor therapeutic effect in a mouse tumor model.

It can be seen that it is still a key issue as to how to further compare and analyze the differences in the expression of CD25 and CD122 receptors on intratumoral Tregs and effector T cells to further improve the efficacy of IL2.

However, in the above-mentioned prior art, the modification of IL2 often requires the introduction of relatively complex biological/chemical synthesis processes, which greatly increases the difficulty and uncertainty of drug development.

### 5. Granulocyte colony stimulating factor (G-CSF)

(1) Febrile Neutropenia (FN) is a highly prevalent side effect of solid tumor chemotherapy.
   Clinically, the incidence of febrile neutropenia (FN) caused by solid tumor chemotherapy is relatively high, and is mainly related to the types of solid tumors and chemotherapy regimens: the incidence of FN in general myelosuppressive chemotherapy regimen is 13%-21%, and the incidence of FN in breast/lung/esophageal/ovarian cancer is significantly higher, reaching 28.1%, 15.6%, 15.6% and 12.5%, respectively. Different chemotherapy regimens result in great differences in the incidence of FN. In particular, the use of taxanes, platinum and fluorouracil is more prone to result in myelosuppression, which is closely related to the incidence of FN.
(2) Granulocyte colony stimulating factor (G-CSF) is a factor that promotes the growth of neutrophils and is widely used to treat FN in clinic.
   It plays an important role in:
   1) supporting the survival of granulopoietic hematopoietic stem cells and stimulating their differentiation into mature neutrophils;
   2) promoting the release of neutrophils from the bone marrow, and enhancing the ability of neutrophils to phagocytize antigens and form oxygen free radicals, which facilitates the elimination of bacteria.
      At present, G-CSF has been widely used in febrile neutropenia (FN) caused by various reasons and has obtained very significant therapeutic effects, especially in FN caused by radiotherapy and chemotherapy.
(3) Product defects of commercially available G-CSF formulations

Similar to interleukin cytokines, there are many different binding modes between G-CSF and its receptor, which is a member of the cytokine receptor superfamily. The initiation of different downstream signaling pathways by G-CSF/G-CSFR binding depends on the structure and function of the intracellular region of the receptor. G-CSFR signaling covers the activation of STAT1, STAT3, and STATS pathways, of which the activated form of STATS is related to the concentration of G-CSF. Protein/receptor binding at different concentrations, in addition to promotion of the growth of neutrophils, may also produce greatly different biological effects, such as stimulation of the proliferation of myeloid-derived suppressor cells (MDSCs) and Tregs.

Despite the significance of G-CSF in the prevention and treatment of FN, G-CSF is grossly underutilized in clinical practice, mainly because the commercially available formulations still have many defects, especially the obvious adverse effects (cutaneous vasculitis) and an increased risk of secondary cancer, in particular:
1) The commercially available formulations (formulas are listed below) have a relatively low pH (4.0), are highly irritating (containing acetic acid and Tween), and are unstable in dilution;
2) Direct exposure of the active ingredient to the injection site activates the release of pro-inflammatory factors (Il-6, and TNF-a);
3) Non-selective activation of cells expressing G-CSF receptors. In addition to activation of myeloid cells to improve FN symptoms, G-CSF promotes the development of myeloid-derived suppressor cells (MDSCs) and the proliferation of Tregs, which in turn promotes tumor progression. Receptors are also expressed in fibroblasts, endothelial cells and even some tumor cells, which is also an important cause of its clinical side effects.

Based on this, the present invention is proposed.

### SUMMARY OF THE INVENTION

The present invention firstly relates to a cytokine protein pharmaceutical formulation, which is a water-soluble formulations, comprising
(1) a cytokine protein with a concentration of 40-20000 µg/ml, preferably with a concentration of 0.1-20 mg/ml, and more preferably with a concentration of 1-10 mg/ml;
(2) polyethylene glycol-distearoyl phosphatidylethanolamine (PEG-DSPE); and
(3) a necessary pH regulator/buffer;

the PEG-DSPE and the cytokine protein have a molar ratio of is 8-100:1; preferably, the PEG-DSPE and the cytokine protein have a molar ratio of 10-80:1; more preferably, the PEG-DSPE and the cytokine protein have a molar ratio of 15-50:1; most preferably, the PEG-DSPE and the cytokine protein have a molar ratio of 20-40:1;
the cytokine protein is a human-derived cytokine, with a structure comprising 3-8 α-helices and a molecular weight of 10-30 kDa;
preferably, the cytokine protein is a human-derived wild-type cytokine protein with a structure only containing α helix/helices,
more preferably, the cytokine protein is a recombinantly expressed cytokine protein with a structure only comprising 4-6 α-helices and a molecular weight of 10-20 KDa;
most preferably, the cytokine is IL2, IL4, IL7, IL9, IL10, IL15, IL21, G-CSF, GM-CSF, IFN, EPO, GF or TNF.

The IL2 protein has an amino acid sequence as shown in SEQ ID NO.1, and the G-CSF protein has an amino acid sequence as shown in SEQ ID NO.2,
SEQ ID NO.1: 
APTSSSTKKTQLQLEHLLLDLQMILNGINNYKNPKLTRMLTFKFYMPKKATELKHLQCLEEELKP
LEEVLNLAQSKNFHLRPRDLISNINVIVLELKGSETTFMCEYADETATIVEFLNRWITFSQSIISTLT
SEQ ID NO.2:

MTPLGPASSLPQSFLLKCLEQVRKIQGDGAALQEKLCATYKLCHPEELVLLGHSLGIPWAPLSSCP
SQALQLAGCLSQLHSGLFLYQGLLQALEGISPELGPTLDTLQLDVADFATTIWQQMEELGMAPALQPT
QGAMPAFASAFQRRAGGVLVASHLQSFLEVSYRVLRHLAQP

the PEG-DSPE has a purity of ≥95%, with a PEG chain having a dispersity of ≤1.1;
preferably, the PEG-DSPE molecule has a PEG chain having a length of 1000-5000 Da; more preferably, the PEG-DSPE molecule has a PEG chain having a length of 1500-2500 Da; most preferably, the PEG-DSPE molecule has a PEG chain having a length of 2000 Da (PEG2000-DSPE).

Preferably, the cytokine protein pharmaceutical formulation is adjusted to have a pH of 5.5-6.0 by using the pH regulator;
the pH regulator/buffer includes, but is not limited to, hydrochloric acid, acetic acid, sodium/potassium hydroxide, sodium bicarbonate, sodium dihydrogen carbonate, sodium hydrogen phosphate, sodium dihydrogen phosphate, and amino acid pH regulators/buffers such as glycine or histidine.

Optionally, the cytokine protein pharmaceutical formulation may be further lyophilized to prepare a lyophilized formulation.

The lyophilized formulation may further comprise a lyoprotectant, including but not limited to glycerin, glycine, sucrose, lactose, albumin, histidine, mannitol or a combination thereof.

The present invention also relates to a method of preparing the cytokine protein pharmaceutical formulation, comprising
(1) preparing an aqueous solution of pH 3.0 to 5.5, preferably by diluting glacial acetic acid to a concentration of 10 mM with deionized water and adjusting the pH to 3.0-4.0, most preferably by diluting glacial acetic acid to a concentration of 10 mM with deionized water and adjusting the pH to 3.0-3.5;
(2) weighing an amount of cytokine protein dry powder, adding the aqueous solution with low pH to the cytokine protein dry powder, and mixing thoroughly to obtain a cytokine protein suspension;
(3) weighing a corresponding amount of PEG-DSPE dry powder according to the amount of the cytokine protein in step (2), adding the PEG-DSPE dry powder to the cytokine protein suspension, and mixing thoroughly;
(4) heating in a water bath at 40°C-60°C for 10-30 minutes;
(5) allowing the solution obtained in step (4) to stand to balance to room temperature, adjusting its pH to 5.5-6.0, and allowing the solution to stand for another10-60 minutes to obtain the cytokine protein formulation;
   the PEG-DSPE has a purity of ≥95%, with a PEG chain having a dispersity of ≤1.1;
   preferably, the PEG-DSPE molecule has a PEG chain having a length of 1000-5000 Da; more preferably, the PEG-DSPE molecule has a PEG chain having a length of 1500-2500 Da; most preferably, the PEG-DSPE molecule has a PEG chain having a length of 2000 Da (PEG2000-DSPE); and
   optionally,
(6) diluting the cytokine protein formulation in the solution obtained in step (5) to an appropriate concentration with the same buffer, or concentrating the cytokine protein formulation in the solution obtained in step (5) by ultrafiltration to an appropriate concentration; and
(7) sterilizing the cytokine protein formulation by filtration.

The cytokine protein pharmaceutical formulation prepared by the method has a protein recovery rate of ≥90%, and the recovery rate is a ratio of the protein content in the cytokine protein pharmaceutical formulation to the added protein amount.

The present invention also relates to use of the cytokine protein pharmaceutical formulation in the manufacture of a medicament; preferably, the medicament is administered by subcutaneous injection.

The present invention also relates to use of polyethylene glycol-distearoyl phosphatidylethanolamine (PEG-DSPE) in the manufacture of a formulation for changing affinity between a protein of interest and its receptor, wherein the protein of interest comprises at least one α-helix; preferably, the protein of interest comprises four to six α-helices; more preferably, the protein of interest is IL2, IL4, IL7, IL9, IL15, IL21, G-CSF or GM-CSF.

Said changing the affinity between the protein of interest and its receptor is reducing binding ability of the protein to a low-affinity receptor; preferably, the low-affinity receptor has an affinity (Kd value) of ≥ 10⁻⁶ M to the protein; more preferably, the low-affinity receptor has an affinity (Kd value) of ≥ 10⁻⁷ M to the protein; and most preferably, the low-affinity receptor has an affinity (Kd value) of ≥ 10⁻⁸ M to the protein.

In the formulation, the PEG-DSPE molecule and the protein of interest have a ratio as follows:
(1) the PEG-DSPE molecule and the α-helix of the protein of interest has a ratio of 2-25: 1 in number; preferably, the PEG-DSPE molecule and the α-helix of the protein of interest has a ratio of 3-20: 1 in number; more preferably, the PEG-DSPE molecule and the α-helix of the protein of interest has a ratio of 4-15: 1 in number; or
(2) the PEG-DSPE and the protein of interest has a molar ratio of 8-100: 1; preferably, the PEG-DSPE and the protein of interest has a molar ratio of 10-80: 1; more preferably, the PEG-DSPE and the protein of interest has a molar ratio of 15-50: 1; most preferably, the PEG-DSPE and the protein of interest has a molar ratio of 20-30: 1.

The PEG-DSPE has a purity of ≥95%, with a PEG chain having a dispersity of ≤1.1; preferably, the PEG-DSPE molecule has a PEG chain having a length of 1000-5000 Da; more preferably, the PEG-DSPE molecule has a PEG chain having a length of 1500-2500 Da; most preferably, the PEG-DSPE molecule has a PEG chain having a length of 2000 Da (PEG2000-DSPE).

The present invention also relates to an IL2/PP formulation, which is a water-soluble formulations, comprising:
(1) IL2 protein with a concentration greater than 1 mg/ml, preferably with a concentration of 1-10 mg/ml;
(2) polyethylene glycol-distearoyl phosphatidylethanolamine (PEG-DSPE); preferably, the PEG-DSPE molecule has a PEG chain having a length of 2000 Da (PEG2000-DSPE), and the PEG-DSPE has a purity of ≥95%, with a PEG chain having a dispersity of ≤1.1; and
(3) a necessary pH regulator/buffer;

the PEG-DSPE and the IL2 protein has a molar ratio of 8-100:1; preferably, the PEG-DSPE and the IL2 protein has a molar ratio of 10-80:1; more preferably, the PEG-DSPE and the IL2 protein has a molar ratio of 15-50:1; most preferably, the PEG-DSPE and the IL2 protein has a molar ratio of 20-30:1;
the IL2 protein is expressed in *Escherichia coli* and purified, and has an amino acid sequence as shown in SEQ ID NO.1;
preferably, the IL2IPP formulation is adjusted to have a pH of 5.5-6.0 by using the pH regulator.

Optionally, the IL2IPP formulation may be further lyophilized to prepare an IL2/PP lyophilized formulation.

The present invention also relates to a method of preparing the IL2IPP formulation, comprising
(1) preparing an aqueous solution with a pH of 3.0-5.5, preferably by diluting glacial acetic acid to a concentration of 10 mM with deionized water and adjusting the pH to 3.0-3.5;
(2) weighing an amount of IL2 protein dry powder, adding the aqueous solution with a pH of 3.0-3.5 to the IL2 protein dry powder , and mixing thoroughly to obtain an IL2 suspension;
(3) weighing a corresponding amount of PEG-DSPE dry powder according to the amount of the IL2 protein in step (2), adding the PEG-DSPE dry powder to the IL2 suspension, and mixing thoroughly;
(4) heating in a water bath at 40°C-60°C for 10-30 minutes;
(5) allowing the solution obtained in step (4) to stand to balance to room temperature, adjusting its pH to 5.5-6.0, and allowing the solution to stand for another 10-60 minutes to obtain the IL2IPP formulation;
   preferably, the PEG-DSPE molecule has a PEG chain having a length of 2000 Da (PEG2000-DSPE), and the PEG-DSPE has a length of ≥95%, with a PEG chain having a dispersity of ≤1.1; and
   optionally,
(6) diluting the IL2 protein in the solution obtained in step (5) to an appropriate concentration with the same buffer, or concentrating the IL2 protein in the solution obtained in step (5) by ultrafiltration to an appropriate concentration;
(7) sterilizing the formulation by filtration.

The IL2/PP formulation prepared by the method has an IL2 protein recovery rate of ≥90%.

The present invention also relates to use of the IL2/PP formulation
(1) in the manufacture of an antitumor medicament;
(2) in the manufacture of a formulation for activating cellular immune response;
(3) in the manufacture of an anti-tumor combined formulation; preferably, the combined formulation further comprises an antibody or an antibody construct, a cytotoxic agent, an immunotherapeutic or cellular therapeutic agent;

the medicament or formulation has lower toxicity and/or side effects compared to the IL2 formulation in the form of a DLS formulation;
said activating cellular immune response refers to, *in vivo* or *in vitro,*
   (1) up-regulating proliferation of CD8+ T cells, and/or
   (2) inducing differentiation of T cells into CD8+ T cells, and/or
   (3) inhibiting differentiation of T cells into Tregs; and/or
   (4) increasing ratio of CD8+T cells/Tregs;
preferably, said activating cellular immune response means that:
   the IL2/PP formulation, compared to the IL2 formulation in the form of a DLS formulation (positive reference formulation) or the formulation in the form of a free-protein solution, is capable *of in vivo* or *in vitro:*
   (1) up-regulating proliferation of CD8+ T cells more, and/or
   (2) inducing differentiation of T cells into CD8+ T cells more, and/or
   (3) inhibiting differentiation of T cells into Tregs more;
   (4) increasing ratio of CD8+T cells/Tregs;
preferably, the medicament or formulation is administered by subcutaneous injection.

The present invention also relates to a G-CSF/PP formulation, which is a water-soluble formulations, comprising:
(1) G-CSF protein with a concentration greater than 1 mg/ml, preferably with a concentration of 1-10 mg/ml;
(2) polyethylene glycol-distearoyl phosphatidylethanolamine (PEG-DSPE); preferably, the PEG-DSPE molecule has a PEG chain having a length of 2000 Da (PEG2000-DSPE), and the PEG-DSPE has a purity of ≥95%, with a PEG chain having a dispersity of ≤1.1; and
(3) a necessary pH regulator/buffer;

the G-CSF protein is expressed in *Escherichia coli* and purified, and has an amino acid sequence as shown in SEQ ID NO.2;
preferably, the G-CSF/PP formulation is adjusted to have a pH of 5.5-6.0 by using the pH regulator; optionally, the G-CSF/PP formulation may be further lyophilized to prepare a G-CSF/PP lyophilized formulation.

The present invention also relates to a method of preparing the G-CSF/PP formulation, comprising
(1) preparing an aqueous solution of pH of 3.0-5.5, preferably by diluting glacial acetic acid to a concentration of 10 mM with deionized water and adjusting the pH to 3.0-3.5;
(2) weighing an amount of G-CSF protein dry powder, adding the aqueous solution with the pH of 3.0-3.5 to the G-CSF protein dry powder, and mixing thoroughly to obtain a G-CSF suspension;
(3) weighing a corresponding amount of PEG-DSPE dry powder according to the amount of G-CSF protein in step (2), then adding the PEG-DSPE dry powder to the G-CSF suspension, and mixing thoroughly;
(4) heating in a water bath at 40°C-60°C for 10-30 minutes;
(5) allowing the solution obtained in step (4) to stand to balance to room temperature, adjusting its pH to 5.5-6.0, and allowing the solution to stand for another 10-60 minutes to obtain the G-CSF/PP formulation;
   preferably, the PEG-DSPE molecule has a PEG chain having a length of 2000 Da (PEG2000-DSPE), and the PEG-DSPE has a purity of ≥95%, with a PEG chain having a dispersity of ≤1.1; and
   optionally,
(6) diluting the G-CSF protein in the solution obtained in step (5) to an appropriate concentration with the same buffer, or concentrating the G-CSF protein in the solution obtained in step (5) by ultrafiltration to an appropriate concentration; and
(7) sterilizing the formulation by filtration.

The G-CSF/PP formulation prepared by the method has a G-CSF protein recovery rate of ≥90%.

The present invention also relates to use of the G-CSF/PP formulation in the manufacture of a medicament for treating febrile neutropenia (FN); preferably, the febrile neutropenia is caused by radiotherapy or chemotherapy of tumors or organ transplantation.

Preferably, the medicament is administered by subcutaneous injection.

Beneficial effects of the invention:
(1) A novel protein assembly is developed by utilizing the highly specific binding of PEG-PE and cytokine protein molecules, and realizes the selective binding of cytokines and their receptor de1 and specific activation of effector cells downstream cytokines by structural remodeling and functional changes of the protein through the interaction between PEG-PE (PP) and cytokines.
(2) The administration route of cytokine pharmaceuticals is improved, and the half-life of pharmaceuticals is prolonged, the toxic and side effects are reduced, and clinical compliance is increased by using formulations for subcutaneous administration.
(3) It is provided a new form of formulation, which significantly improves the solubility and stability of cytokine pharmaceuticals, and prolongs the *in vivo* half-life thereof.

In summary, the present invention provides a novel and feasible clinical medication solution for cytokine pharmaceuticals, which can overcome the main defects in clinical use and achieve better therapeutic effect, and greatly expand the scope of the clinical application of cytokine drugs.

### BRIEF DESCRIPTION OF THE DRAWINGS

Fig. 1. Schematic representation of IL-2 binding to low-affinity, medium-affinity and high-affinity IL-2 receptors [13].
Fig. 2. Fig. 2A, a photo of a finished IL2/PP formulation; Fig. 2B, a photo of a finished G-CSF/PP formulation.
Fig. 3. Fig. 3A, biological activity of the IL2/PP formulation and the positive reference DLS determined by CTLL-2/MTT assay; Fig. 3B, biological activity of the G-CSF/PP formulation determined by NFS-60 cell proliferation assay.
Fig. 4. Interaction between IL-2 and PEG2000-DSPE and SDS determined by isothermal titration calorimetry (ITC).
Fig. 4A. Results of IL-2 and SDS titration, performed at an experimental temperature of 25°C;
Fig. 4B. Results of IL-2 and PEG2000-DSPE titration, performed at an experimental temperature of 25°C;
Fig. 4C. Results of IL-2 and PEG2000-DSPE titration, performed at an experimental temperature of 50°C.
Fig. 5. Inhibition of tumor development by the IL2/PP formulation and the positive reference DLS evaluated and compared in a CT26-Balb/C mouse tumor-bearing model (peritoneal + subcutaneous administration).
Fig. 5A. Changes in the tumor volume of mice in individual groups during the whole evaluation period (mean ± SEM);
Fig. 5B. Changes in the body weight of mice in individual groups from the beginning of administration to one week after the completion of administration (mean ± SEM).
Fig. 6. Inhibition of tumor development by the IL2/PP formulation and the positive reference DLS observed and compared in a CT26-Balb/C mouse tumor-bearing model (tail vein & subcutaneous administration).
Fig. 6A. Changes in the tumor volume of mice in individual groups during the whole evaluation period (mean ± SEM);
Fig. 6B. Changes in the body weight of mice in individual groups during the whole evaluation period (mean± SEM);
Fig. 6C. Differences in the survival of mice in individual groups (in addition to the actual dead mice/mouse, the mouse was deemed as the dead when the tumor volume > 2000 mm³).
Fig. 7. Comparison of the toxic and side effects induced by continuous administration of the II,2/PP formulation and the positive reference DLS in a CT26-Balb/C mouse tumor-bearing model (tail vein & subcutaneous administration).
Fig. 7A. After continuous subcutaneous administration of the positive reference DLS, a visible epidermal ulcer appeared at the injection site and scabbed at the wound (scabbed ulcer of needle-size);
Fig. 7B. After continuous subcutaneous administration of the IL2/PP formulation, no obvious adverse effects appeared, and the injection site was completely in a normal state;
Fig. 7C. After continuous tail vein administration of the positive reference DLS, severe ulcer, congestion and swelling appeared at the whole tail of the mouse, which not only made it difficult to complete the injection, but also resulted in very slow recovery after the administration was stopped;
Fig. 7D. After continuous tail vein administration of the IL2/PP formulation, no obvious adverse effects appeared, and the entire tail was completely in a normal state, allowing the completion of the continuous injection;
Fig. 7E. The tail of a mouse with severe toxic and side effects in the group of tail vein administration of the positive reference DLS gradually became necrotic after the hematoma was ruptured, and finally broke off.
Fig. 8. Inhibition of tumor development by the IL2/PP formulation and the positive reference DLS evaluated and compared in a B16F10-C57BL/6 mouse tumor-bearing model (tail vein & subcutaneous administration).
Fig. 8A. Changes in the tumor volume of the 4 groups of mice administered by subcutaneous injection during the whole evaluation period (mean ± SEM);
Fig. 8B. Changes in the tumor volume of the two groups of mice administered by tail vein injection during the whole evaluation period (mean ± SEM);
Fig. 8C. Changes in the tumor volume of the three groups of mice administered with the DLS formulation during the whole evaluation period (mean ± SEM);
Fig. 8D. Changes in the tumor volume of the three groups of mice administered with the IL2/PP formulation during the whole evaluation period (mean ± SEM);
Fig. 8E. Changes in the body weight of mice in individual groups during the whole evaluation period (mean ± SEM).
Fig. 9. Comparison of the toxic and side effects induced by continuous administration of the IL2/PP formulation and the positive reference DLS in a B16F10-C57NL/6 mouse tumor-bearing model (tail vein & subcutaneous administration).
Fig. 9A. After continuous subcutaneous administration of the positive reference DLS, a visible epidermal ulcer appeared at the injection site and scabbed at the wound, and the wound gradually healed 4 to 6 days after the administration was stopped;
Fig. 9B. After continuous subcutaneous administration of the IL2IPP formulation, no obvious adverse effects appeared, and the injection site was completely in a normal state;
Fig. 9C. After continuous tail vein administration of the positive reference DLS, severe congestion and swelling appeared throughout the tail of the mouse and an ulcer appeared at the injection site, which made it difficult to complete the injection, and the wound gradually recovered after about one week after administration was stopped;
Fig. 9D. After continuous tail vein administration of the IL2IPP formulation, no obvious adverse effects appeared, and the whole tail was completely in a normal state;
Fig. 9E. The ulceration and scabbing site of a mouse with severe toxic and side effects in the group of subcutaneous administration of the positive reference DLS was not completely healed 2 weeks after administration was stopped;
Fig. 9F. The ulceration site of the tail of a mouse with severe toxic and side effects in the group of tail vein administration of the positive reference DLS did not recover 2 weeks after administration was stopped.
Fig. 10. Selective effects of the IL2/PP formulation and the positive reference DLS on different T cell subsets evaluated and compared in a CT26-Balb/C mouse tumor-bearing model.
Fig. 10A. The percentage of activated immunosuppressive Tregs (CD45+CD4+Foxp3+) and CD8+ effector T cells (CD45+CD3+CD8+) in the spleen of tumor-bearing mice induced by the IL2/PP formulation and the positive reference DLS and the ratio of the two cells (mean + SEM) after continuous intraperitoneal administration;
Fig. 10B. The percentage of activated immunosuppressive Tregs (CD45+CD4+Foxp3+) and CD8+ effector T cells (CD45+CD3+CD8+) in the tumor tissues of tumor-bearing mice induced by the IL2/PP formulation and the positive reference DLS and the ratio of the two cells (mean ± SEM) after continuous intraperitoneal administration.
Fig. 11. Interaction between G-CSF and PEG2000-DSPE determined by isothermal titration calorimetry (ITC).
Fig. 11A. Results of G-CSF and PEG2000-DSPE titration, performed at an experimental temperature of 25°C;
Fig. 11B. Results of G-CSF and PEG2000-DSPE titration, performed at an experimental temperature of 50°C.
Fig. 12. The results of the pharmacodynamic study of G-CSF/PP in tumor-bearing mice show that G-CSF/PP had a similar leukocyte elevation effect to the G-CSF protein bulk, significantly reduced the number of MDSCs and Tregs and effectively reduced the immune-related side effects of G-CSF.

### DETAILED DESCRIPTION OF THE EMBODIMENTS

### Materials and methods

IL-2 protein and G-CSF protein were expressed in *Escherichia coli* and purified, and have an amino acid sequence as shown in SEQ ID NO.1 and SEQ ID NO.2, respectively:
SEQ ID NO.1 (IL2): 
APTSSSTKKTQLQLEHLLLDLQMILNGINNYKNPKLTRMLTFKFYMPKKATELKHLQCLEEELKP
LEEVLNLAQSKNFHLRPRDLISNINVIVLELKGSETTFMCEYADETATIVEFLNRWITFSQSIISTLT
SEQ ID NO 2 (G-CSF):

MTPLGPASSLPQSFLLKCLEQVRKIQGDGAALQEKLCATYKLCHPEELVLLGHSLGIPWAPLSSCP
SQALQLAGCLSQLHSGLFLYQGLLQALEGISPELGPTLDTLQLDVADFATTIWQQMEELGMAPALQPT
QGAMPAFASAFQRRAGGVLVASHLQSFLEVSYRVLRHLAQP

PEG₂₀₀₀-DSPE (polyethylene glycol2000-distearoyl phosphatidylethanolamine) was purchased from Lipoid Group.

### Positive reference formulation

ADLS formulation was used as a positive reference formulation, which is an IL2 protein formulation containing SDS (sodium dodecyl sulfate) as a co-solvent. Its composition and preparation are prior art and well known to those skilled in the art, see, for example, EP1688146B1, etc.

### Cell lines:

CTLL-2: ATCC Number TIB-214, a cytotoxic T lymphocyte derived from C57BL/6 strain mice, its normal growth and proliferation depending on a certain concentration of IL-2, commonly used as a reporter cell to evaluate the biological activity of IL-2 and analogues thereof.

Murine colon carcinoma cell CT26: ATCC Number CRL-2638, derived from Balb/c strain mice, an undifferentiated murine colon carcinoma cell induced by N-nitroso-N-methylurethane (NNMU), exhibiting fibroblast-like morphology, growing adherently, commonly used to test immunotherapy protocols or in studies on the host immune response.

Murine skin melanoma cell B16F10: ATCC Number CRL-6475, derived from the skin of C57BL/6J strain melanoma tumor-bearing mice, a subline of B16F0, exhibiting fibroblast-like morphology, growing adherently, commonly used to construct a mouse melanoma animal model.

### Cell line culture method:

Culture of mouse IL-2-dependent cytotoxic T lymphocyte CTLL-2 with RPMI-1640 medium supplemented with 10% fetal bovine serum, 400-800 IU/ml rhIL-2 and 50 µM β-mercaptoethanol. The frozen cells were taken out of the liquid nitrogen storage tank and immediately put into a 37°C water bath for rapid thawing. The cell suspension was then transferred into a centrifuge tube containing 10 ml of the culture medium, and centrifuged at 1500 × g for 5 min to remove the supernatant. After re-suspension with fresh medium, the cells were transferred to a cell culture flask containing 10-15 ml of the culture medium, and cultured in a 37°C incubator with a humidified atmosphere of 5% CO₂. The cells were observed every day or every other day, replaced with fresh medium in time and passaged.

Culture of the CT26 cell line for murine colon carcinoma model with RPMI-1640 medium (containing 10% fetal bovine serum). The frozen cells were taken out of the liquid nitrogen storage tank and immediately put into a 37°C water bath for rapid thawing. The cell suspension was then transferred into a centrifuge tube containing 10 ml of culture medium, and centrifuged at 700 × g for 5 min to remove the supernatant. After re-suspended with fresh medium, the cells were transferred to a cell culture flask containing 10-15 ml of culture medium and cultured in a 37°C incubator with a humidified atmosphere of 5% CO₂. The cells were observed every day or every other day, replaced with fresh medium in time and passaged.

Culture of mouse melanoma B16F10 cell line with DMEM medium (containing 10% fetal bovine serum). The frozen cells were taken out of the liquid nitrogen storage tank and immediately put into a 37°C water bath for rapid thawing. The cell suspension was then transferred into a centrifuge tube containing 10 ml of culture medium, and centrifuged at 700 × g for 5 min to remove the supernatant. After re-suspended with fresh medium, the cells were transferred to a cell culture flask containing 10-15 ml of the culture medium and cultured in a 37°C incubator with a humidified atmosphere of 5% CO₂. The cells were observed every day or every other day, replaced with fresh medium in time and passaged.

### Laboratory animals:

BALB/c: SPF grade, 6-8 weeks old, female, purchased from Beijing Vital River Laboratory Animal Technology Co., Ltd., in line with the Beijing laboratory animal quality standards.

C57BL/6N: SPF grade, 6-8 weeks old, female, purchased from Beijing Vital River Laboratory Animal Technology Co., Ltd., in line with the Beijing laboratory animal quality standards.

### Example 1. Preparation of IL2/PP formulation and G-CSF/PP formulation

### 1. Preparation of IL2/PP formulation

(1) Glacial acetic acid was diluted with deionized water to a concentration of 10 mM and a pH of 3.0-3.5, filtered by a 0.22 µm filter membrane and used as a solvent.
(2) An amount of IL-2 protein lyophilized powder was weighed, added with the 10 mM diluted acetic acid solution, and mixed thoroughly to obtain an IL-2 suspension.
(3) A corresponding amount of PEG₂₀₀₀-DSPE powder was weighed according to the amount of the IL2 in step (2) to make the molar ratio of the two reach 15-30:1 (PEG₂₀₀₀-DSPE: IL2), added to the IL2 suspension, and mixed thoroughly.
(4) The mixture was heated in a water bath at 40°C-60°C for 10-30 minutes.
(5) The sample was allowed to stand to balance to room temperature.
(6) The sample was adjusted to have a pH of 5.5-6.0 by adding 1 M NaOH solution, and allowed to stand for another 10-60 minutes.
(7) The obtained sample was diluted to an appropriate concentration with a solvent having the same salt concentration and pH.
(8) The sample was filtered with a 0.22 µm filter membrane to obtain a IL2/PP formulation.

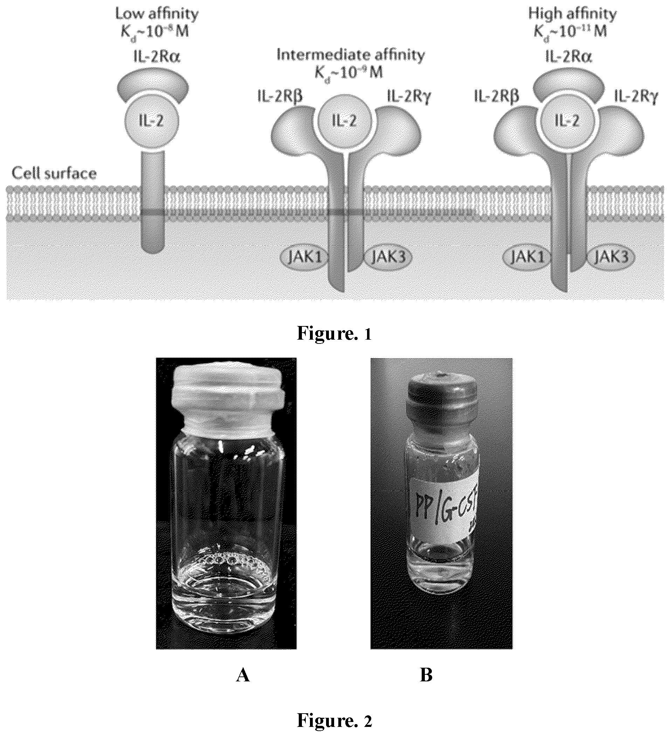

The filtered product was detected by BCA for protein concentration, and then sealed and stored at 4°C. The obtained IL2/PP formulation had an appearance as shown in Fig. 2A, and was a colorless, clear and transparent solution without any visible insolubles.

### 2. Preparation of G-CSF/PP formulation

(1) Glacial acetic acid was diluted with deionized water to a concentration of 10 mM and a pH of 3.0-3.5, filtered by a 0.22 µm filter membrane and used as a solvent.
(2) An amount of G-CSF protein lyophilized powder was weighed, added with the 10 mM diluted acetic acid solution, and mixed thoroughly to obtain a G-CSF suspension.
(3) A corresponding amount of PEG2000-DSPE powder was weighed according to the amount of the G-CSF in step (2) to make the molar ratio of the two reach 15-30:1 (PEG₂₀₀₀-DSPE: IL2), added to the G-CSF suspension, and mixed thoroughly.
(4) The mixture was heated in a water bath at 40°C-60°C for 10-30 minutes.
(5) The sample was allowed to stand to balance to room temperature.
(6) The sample was adjusted to have a pH of 5.5-6.0 by adding 1 M NaOH solution, and allowed to stand for another 10-60 minutes.
(7) The obtained sample was diluted to an appropriate concentration with a solvent having the same salt concentration and pH.
(8) The sample was filtered with a 0.22 µm filter membrane to obtain a G-CSF/PP formulation.

The filtered product was detected by BCA for protein concentration, and then sealed and stored at 4°C. The obtained G-CSF/PP formulation had an appearance as shown in Fig. 2B, and was a colorless, clear and transparent solution without any visible insolubles.

### Example 2. Determination of biological activity of IL2/PP formulation and G-CSF/PP formulation by cell assay

### 1. Determination of biological activity of IL2/PP formulation by CTLL-2/MTT cell proliferation assay

### Reagents:

(1) Basal medium: RPMI 1640 + 10% FBS + 100 U/ml penicillin + 100 U/ml streptomycin.
(2) Complete medium: basal medium supplemented with IL2 at a final concentration of 400-800 IU/ml.
(3) PBS: containing 8 g NaCl, 0.20 g KCl, 1.44 g Na₂HPO₄, and 0.24 g KH₂PO₄ per liter, autoclaved at 121°C for 15min.
(4) Methyl thiazolyl tetrazolium solution (MTT): at a concentration of 5 mg/ml, filtered by a 0.22 µm filter membrane for sterilization, and stored at 4°C in the dark.
(5) Lysis solution: 15% sodium dodecyl sulfate solution (SDS).

### Experimental protocols:

(1) Preparation of samples: a positive reference, DLS, with known biological activity was used as the standard, and the IL2/PP formulation obtained from Example 1 was used as the test sample. The above two formulations were diluted with basal medium to an appropriate concentration, for example, 400-800 IU/ml.
(2) Preparation of CTLL-2 cell suspension: CTLL-2 cells that were cultured in the complete medium and grew productively after thawing were washed 3 times with 1640 culture medium without other ingredients, and then re-suspended with the basal medium to prepare a cell suspension with an appropriate concentration, for example, 2-6 ×10⁵/ml.
(3) Addition of samples: A flat-bottomed 96-well plate was taken, the test samples and the standards were gradient diluted with the basal medium to obtain at least 8 concentrations of samples which differ from each other by a factor of 2 or more, and the samples of each concentration were added into 2 to 3 wells with 50 µl per well.
(4) Cell seeding: 50 µl of cell suspension was added to each well, mixed thoroughly and placed in a 37°C cell incubator with 5% CO₂ for 18-24 hours.
(5) Each well was added with 20 µl MTT solution and then incubated in the incubator for 4-6 hours.
(6) Each well was added with 150 µl of 15% SDS solution, mixed thoroughly, and then incubated in the incubator for 18-24 hours.
(7) The absorbance at 570 nm was determined by using a microplate reader, and the IL-2 activity of the test samples was calculated according to the following method.

**Specific activity calculation:** biological activity (IU/ml) of the test sample =Pr × (Ds × Es) / (Dr × Er), wherein,
Pr: biological activity of the standard, IU/ml
Ds: pre-dilution factor of the test sample
Dr: pre-dilution factor of the standard
Es: dilution factor of the test sample equivalent to the median effective dose of the standard
Er: dilution factor of the median effective dose of the standard

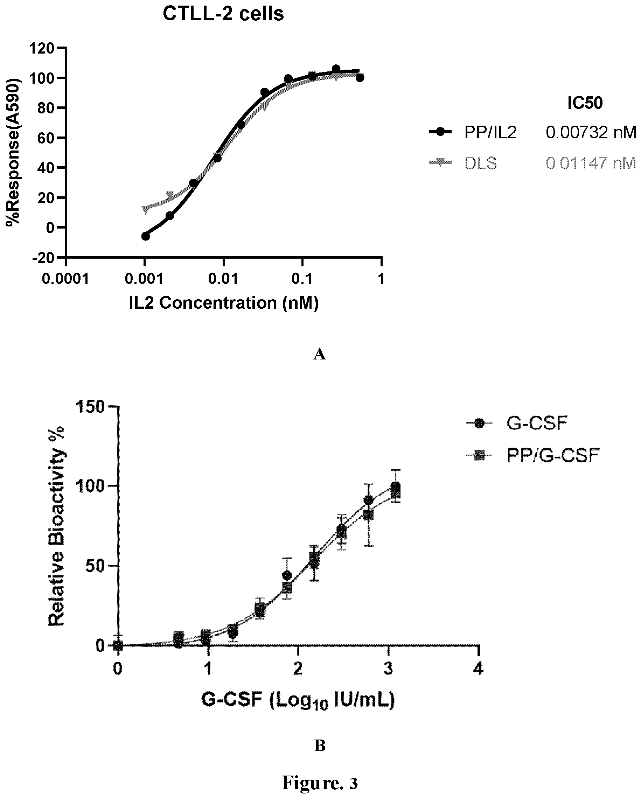

The experimental results **(****Fig. 3A****)** showed that,
(1) the biological activity of the IL-2 protein molecule in the IL2/PP formulation was substantially the same as that of the protein molecule in the positive reference (DLS), even slightly higher than that of DLS;
(2) the half-maximal inhibitory concentration (IC₅₀) of the IL2/PP was about 0.00723 mM, which is significantly lower than the IC₅₀ of DLS (0.01147 nM).

### 2. Determination of biological activity of G-CSF/PP formulation by NFS-60 cell proliferation assay

### Reagents:

(1) Basal medium: RPMI 1640 + 10% FBS + 100 U/ml penicillin + 100 U/ml streptomycin.
(2) Complete medium: basal medium supplemented with 5.5 mM β-mercaptoethanol and 62 ng/mL M-C SF.
(3) PBS: containing 8 gNaCl, 0.20 gKCl, 1.44 gNa₂HPO₄, and 0.24 g KH₂PO₄ per liter, autoclaved at 121°C for 15min.
(4) Methyl thiazolyl tetrazolium solution (MTT): at a concentration of 5 mg/ml, filtered by a 0.22 µm filter membrane for sterilization, and stored at 4°C in the dark.
(5) Lysis solution: 10% Triton X-100, 2.8% concentrated hydrochloric acid, and 87.2% isopropanol.

### Experimental protocols:

(1) Preparation of samples: a positive reference, SH protein bulk, with known biological activity was used as the standard, and the G-CSF/PP formulation obtained from Example 1 was used as the test sample. The above two formulations were diluted with basal medium to an appropriate concentration, for example, 0-120 IU/ml.
(2) Preparation of NFS-60 cell suspension: NFS-60 cells that were cultured in the complete medium and grew productively after thawing were washed 3 times with the basal medium, and then re-suspended with the basal medium to prepare a cell suspension with a density of 2×10⁵/ml.
(3) Cell seeding: Cells were seeded in a flat-bottomed 96-well plate by adding 50 µL of the cell suspension (containing 1×10⁴ cells) to each well, and then were allowed to grow overnight and added with the sample the next day.
(4) Addition of samples: A flat-bottomed 96-well plate was taken, test samples and the standards were gradient diluted with the basal medium to obtain at least 8 concentrations of samples which differ from each other by a factor of 2 or more, and the samples of each concentration were added into 2 wells with 50 µl per well. The cells were incubated in the incubator for 48 hours.
(5) Each well was added with 20 µl of the MTT solution and then incubated in the incubator for 4- 6 hours.
(6) Each well was added with 100 µl of the lysis solution and mixed thoroughly. The absorbance at 570 nm was then determined by using a microplate reader with 630 nm as the reference wavelength and the measurement results were recorded. The test data were processed by a computer program or the four-parameter regression calculation method, and the G-CSF activity of the test sample was calculated according to the following method.

Specific activity calculation: biological activity (IU/ml) of the test sample =Pr × (Ds × Es) / (Dr × Er), wherein,
Pr: biological activity of the standard, IU/ml
Ds: pre-dilution factor of the test sample
Dr: pre-dilution factor of the standard
Es: dilution factor of the test sample equivalent to the median effective dose of the standard
Er: dilution factor of the median effective dose of the standard

The experimental results (Fig. 3A) showed that,
(1) the biological activity of the G-CSF protein molecule in the G-CSF/PP formulation was substantially the same as that of the protein molecule in the positive reference (SH);
(2) the half-maximal effective concentration (EC50) of the G-CSF/PP was about 146.0 IU/mL, which was not significantly different from that of the positive reference (SH) with an EC₅₀ of 149.4 IU/mL.

### Example 3. Determination of the affinity of interaction between the protein of interest and PEG2000-DSPE

### 1. Determination of the affinity of interaction between IL2 and PEG2000-DSPE and SDS

### (1) Preparation of samples:

A. PEG₂₀₀₀-DSPE solution: dissolved in 10 mM sodium acetate buffer (pH=5.5), with a concentration of 0.5 mM;
B. SDS solution: dissolved in 7.69 mM sodium phosphate buffer (pH=7.6), with a concentration of 1.73 mM;
C. IL-2 solution: dissolved in 10 mM sodium acetate buffer (pH=5.5), with a concentration of 0.005 mM;
D. IL-2 solution: dissolved in 7.69 mM sodium phosphate buffer (pH=7.0), with a concentration of 0.0164 mM.

### (2) Parameter settings and experimental conditions:

The MicroCal ITC 200 isothermal titration calorimeter and its supporting operating software from Malvern Panalytical Company were used. Refer to the table below for specific parameter settings:

| Experiment Parameters | |
|---|---|
| Total number of titration | 20 |
| Temperature of sample cell | 25°C or 50°C |
| Reference power | 8 µcal/sec |
| Initial delay | 60 sec |
| Speed of stirring | 750 RPM |
| Volume per titration | 2.0 µl |
| Duration of titration | 4.0 sec |
| Spacing | 120 sec |

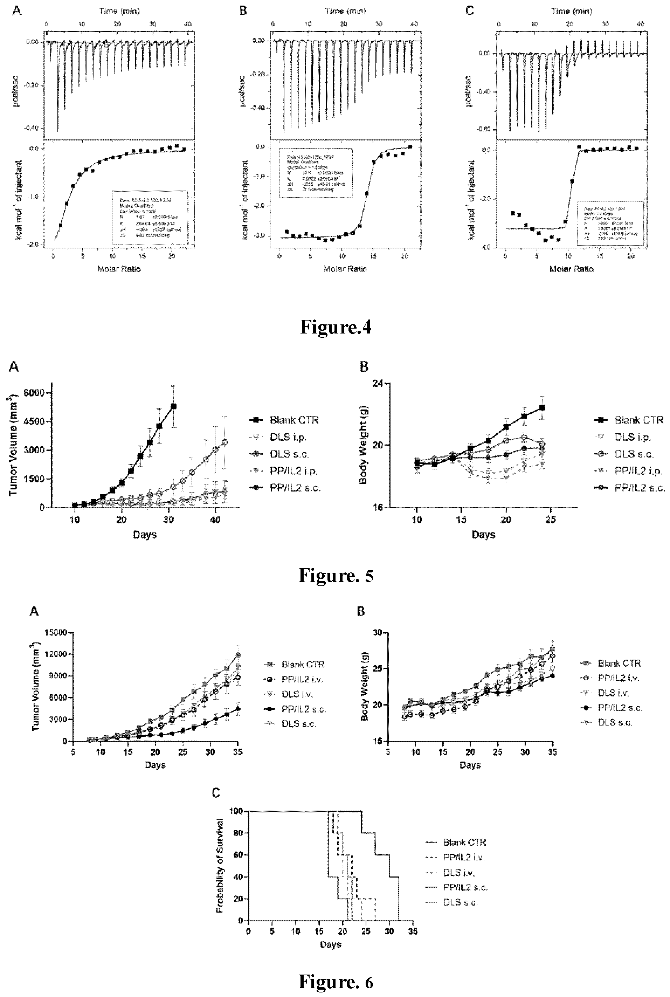

(3) To determine the interaction between PEG2000-PE and IL-2, 40 µl sample A was added to the syringe, and 250 µl sample C was added to the sample cell. To determine the interaction between SDS and IL-2, 40 µl sample B was added to the syringe, and 250 µl sample D was added to the cell. The titration was started after the corresponding sample concentration and reaction temperature were set in the software. After the reaction was completed, the measured heat change was integrated and subjected to reaction curve fitting **(****Fig. 4****)** to calculate various parameters of the interaction between the two samples, wherein the K value measured in the ITC is the binding constant, with a unit of M⁻¹, and its reciprocal is the dissociation constant Kd of the two substances, with a unit of M. The smaller the Kd value, the stronger the affinity between the two substances.

(4) The results showed that the interaction between IL-2 protein and SDS was very weak (the calculated equilibrium dissociation constant Kd was about 3.73 × 10⁻⁵ M). In contrast, IL-2 and PEG₂₀₀₀-DSPE could spontaneously interact with each other under this condition (Kd was about 1.17×10⁻⁷ M), and the affinity therebetween was about two orders of magnitude higher than that between the IL-2 protein and SDS. The affinity between IL-2 and PEG₂₀₀₀-DSPE increased significantly (Kd was about 1.28×10⁻⁸ M) with increase of the reaction temperature (from 25°C to 50°C). These results indicate that the interaction between IL-2 protein and PEG₂₀₀₀-DSPE is much stronger than that between IL-2 protein and SDS in the positive reference under the preparation conditions described in Example 1.

### 2. Determination of the affinity of the interaction between G-CSF and PEG2000-DSPE

(1) Preparation of samples:
A. PEG2000-DSPE solution: dissolved in 10 mM sodium acetate buffer (pH=3.5), with a concentration of 1.24 mM;
B. G-CSF solution: dissolved in 10 mM sodium acetate buffer (pH=3.5), with a concentration of 0.0062 mM;

(2) Parameter settings and experimental conditions:
The MicroCal ITC 200 isothermal titration calorimeter and its supporting operating software from Malvern Panalytical Company were used. Refer to the table below for specific parameter settings:

| Experiment Parameters | |
|---|---|
| Total number of titration | 20 |
| Temperature of sample cell | 25°C or 50°C |
| Reference power | 8 ucal/sec |
| Initial delay | 60 sec |
| Speed of stirring | 750 RPM |
| Volume per titration | 2.0 ul |
| Duration of titration | 4.0 sec |
| Spacing | 120 sec |

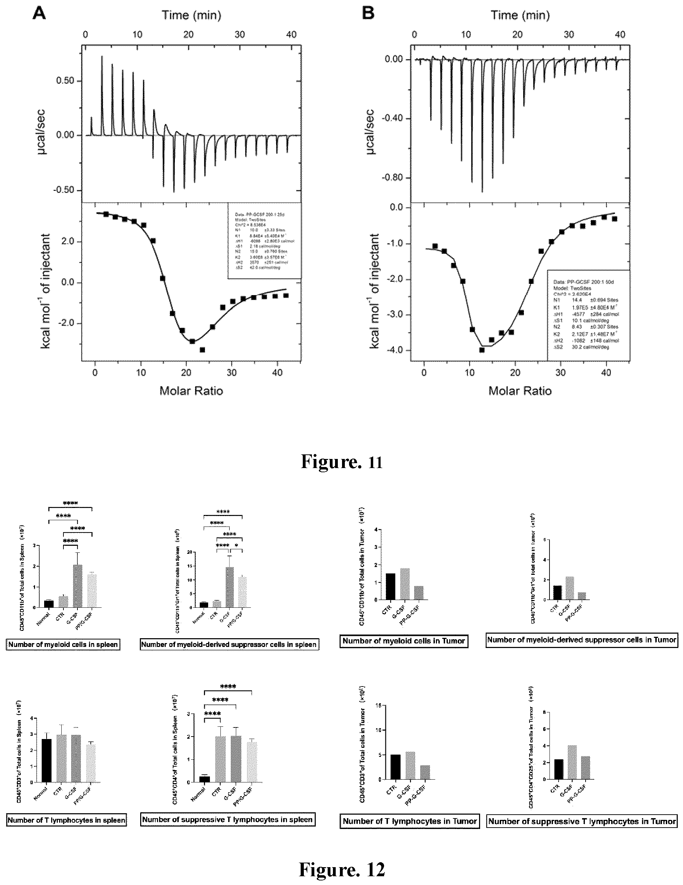

(3) To determine the interaction between PEG₂₀₀₀-PE and G-CSF, 40 µl sample A was added to the syringe, and 250 µl sample B was added to the sample cell. The titration was started after the corresponding sample concentration and reaction temperature were set in the software. After the reaction was completed, the measured heat change was integrated and subjected to reaction curve fitting **(****Fig. 11****)** to calculate various parameters of the interaction between the two samples, wherein the K value measured in the ITC is the binding constant, with a unit of M⁻¹, and its reciprocal is the dissociation constant Kd of the two substances, with a unit of M. The smaller the Kd value, the stronger the affinity between the two substances.
(4) The results showed that G-CSF and PEG2000-DSPE had a very high affinity under this condition (Kd was about 2.78×10⁻⁷ M), and the titration curve showed a typical dual binding site pattern, indicating that there are two or even more high-affinity interaction sites or regions therebetween. The affinity between G-CSF and PEG₂₀₀₀-DSPE also increased significantly (Kd was about 4.72×10⁻⁸ M) with increase of the reaction temperature (from 25°C to 50°C). These results indicates that a very strong interaction could spontaneously occur between the G-CSF protein and PEG₂₀₀₀-DSPE under the preparation conditions described in Example 1.

### Example 4. Determination of Functional activity of IL2/PP formulation in the CT26-Balb/C mouse tumor-bearing model

### Experimental protocols (1): Intraperitoneal administration & subcutaneous administration

(1) CT26 cells thawed from liquid nitrogen were cultured in RPMI-1640 medium (containing 10% fetal bovine serum) for 5-7 days, and passaged more than 3 times in order to ensure that the cells are in a normal state before use. On the day of the experiment, cells should grow to 80%-90% confluence and be in a good state. The cells were digested with 0.05% trypsin, neutralized with 10 times volume of the fresh medium, centrifuged at 700×g to discard the supernatant, re-suspended in an appropriate volume of sterile PBS and counted. The cell suspension was adjusted to a density of 5 ×10⁶/ml with sterile PBS and placed on ice for later use.
(2) The female wild-type Balb/C mice aged 6-8 weeks were inoculated with the CT26 cell suspension subcutaneously on the right back by using a 1 ml sterile syringe. Each mouse was injected with 0.1 ml of the CT26 cell suspension, that is, 5 ×10⁵ CT26 cells.
(3) On day 10 after inoculation, the mice had a visible tumor with a volume of 120±10 mm³ growing at the inoculation site. Mice with tumors of appropriate size and shape were grouped, so that the distribution and the mean value of the tumor volume in individual groups were substantially the same. A total of 5 experimental groups were set up, with 8 mice in each group:
   A. Blank control group (Blank CTR)
   B. Positive reference DLS, intraperitoneal administration group (DLS i.p.)
   C. Positive reference DLS, subcutaneous administration group (DLS s.c.)
   D. IL2/PP formulation, intraperitoneal administration group (IL2/PP i.p.)
   E. IL2/PP formulation, subcutaneous administration group (IL2/PP s.c.)
(4) Starting from day 11 after inoculation, each mouse was injected with 0.1 ml of the corresponding formulation (the actual dosage of IL-2 per injection was 10 µg) twice a day for 8 days, with a total of 16 administrations, according to the administration route of the group to which the mouse belongs (the blank control group did not receive any treatment).
(5) The IL2IPP formulation was prepared according to the method described in Example 1. The lyophilized powder of the positive reference DLS was reconstituted with water for injection to prepare the injection. Both were quantified by BCA and adjusted to the required concentration.
(6) The state of the mice in each group was continuously observed from the completion of grouping. The tumor volume and body weight changes of the tumor-bearing mice were measured three times a week, and the death was recorded.
(7) The experimental results **(****Fig. 5****)** showed that, under the treatment of this administration regimen,
   ① when administered by intraperitoneal injection, both IL2/PP and DLS formulations significantly inhibited the growth of tumor and controlled the tumor volume within a small size compared with the blank control group, but there was no significant difference between the two groups,
   ② when administered by subcutaneous injection, both IL2/PP and DLS formulations significantly inhibited the growth of tumor compared with the blank control group, and there was also a significant difference between the two groups, that is, the inhibitory effect of the IL2IPP formulation group was significantly better than that of the DLS formulation group, and reached the same level of inhibition as in the intraperitoneal administration group, which suggests that the IL2IPP formulation has superior activity and efficacy than the positive reference DLS;
   ③the changes in the body weight showed that, after the administration was started, the mice in the intraperitoneal administration group experienced a rapid and substantial weight loss, accompanied by a decrease in the mouse activity and body temperature and a poor survival state, which gradually recovered after the administration was completed. In contrast, the body weight of the mice in the subcutaneous administration group continued to increase, and the state of the mice remained stable. These results suggest that although intraperitoneal administration has advantages such as fast-absorption and good efficacy, it also has strong toxic and side effects, which will seriously affect the survival of mice, while subcutaneous administration is relatively safer.

In summary, the IL2IPP formulation was able to balance therapeutic effect and safety when administered subcutaneously, and is overall superior to the prior-art positive reference DLS.

### Experimental protocols (2): Tail vein administration & subcutaneous administration

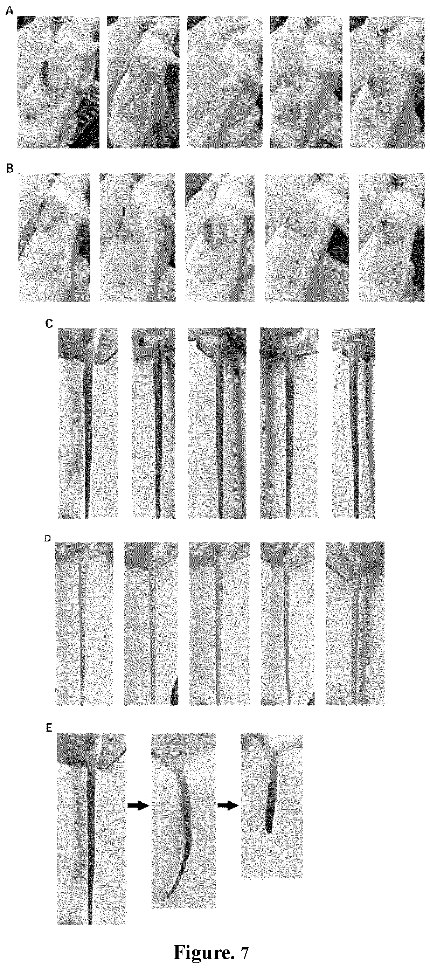

(1) CT26 cells thawed from liquid nitrogen were cultured in RPMI-1640 medium (containing 10% fetal bovine serum) for 5-7 days, and passaged more than 3 times in order to ensure that the cells are in a normal state before use. On the day of the experiment, cells should grow to 80%-90% confluence and be in a good state. The cells were digested with 0.05% trypsin, neutralized with 10 times volume of the fresh medium, centrifuged at 700×g to discard the supernatant, re-suspended in an appropriate volume of sterile PBS and counted. The cell suspension was adjusted to a density of 5 ×10⁶/ml with sterile PBS and placed on ice for later use.
(2) The female wild-type Balb/C mice aged 6-8 weeks were inoculated with the CT26 cell suspension subcutaneously on the right back by using a 1 ml sterile syringe. Each mouse was injected with 0.1 ml of the CT26 cell suspension, that is, 5×10⁵ CT26 cells (the cell suspension should be mixed thoroughly before each pipetting).
(3) On day 8 after inoculation, the mice had a visible tumor with a volume of 150±10 mm³ growing at the inoculation site. Mice with tumors of appropriate size and shape were grouped, so that the distribution and the mean value of the tumor volume in each group were substantially the same. A total of 5 experimental groups were set up, with 5 mice in each group:
   A. Blank control group (Blank CTR)
   B. Positive reference DLS, tail vein administration group (DLS i.v.)
   C. Positive reference DLS, subcutaneous administration group (DLS s.c.)
   D. IL2/PP formulation, tail vein administration group (IL2/PP i.v.)
   E. IL2/PP formulation, subcutaneous administration group (IL2/PP s.c.)
(4) Starting from day 9 after inoculation, each mouse was injected with 0.1 ml of the corresponding formulation (the actual dosage of IL-2 was 16 µg) once a day for 8 days according to the administration route of the group to which the mouse belongs (the blank control group did not receive any treatment).
(5) The IL2IPP formulation was prepared according to the method described in Example 1. The lyophilized powder of the positive reference DLS was reconstituted with water for injection to prepare the injection. Both were quantified by BCA and adjusted to the required concentration.
(6) The state of the mice in each group was continuously observed from the completion of grouping. The tumor volume and body weight changes of the tumor-bearing mice were measured three times a week, the death was recorded, and the toxic and side effects on the mice in each group were observed during the administration.
(7) The experimental results (Fig. 6 and Fig. 7) showed that, under the treatment of this administration regimen,
   ① when administered by subcutaneous injection, the IL2/PP formulation significantly inhibited the growth and development of tumors and prolonged the survival of the tumor-bearing mice compared with the blank control group, while the DLS formulation had a much weaker effect and only slightly improved tumor volume growth;
   (2) when administered by tail vein injection, the IL2IPP and DLS formulations had an inhibitory effect which was not significant compared with the blank control group;
   (3) when treated with the IL2/PP formulation at the same dose, the subcutaneous administration had significantly better effects than the tail vein administration, and there was a significant difference between the two administration routes;
   ④when treated with the DLS formulation at the same dose, the subcutaneous administration and the tail vein administration had substantially the same but not significant effects;
   ⑤when administered continuously by either subcutaneous injection or tail vein injection, the IL2IPP formulation was much safer than the DLS formulation and has almost no adverse effects, while the DLS formulation had very significant and serious side effects.

In summary, when administered by subcutaneous injection, the IL2IPP formulation was significantly better than the prior-art positive reference DLS in enhancing the anti-tumor effect, prolonging the survival, and improving safety.

### Example 5. Determination of functional activity of IL2/PP formulation in B16F10-C57BL/6 mouse tumor-bearing model (tail vein administration & subcutaneous administration)

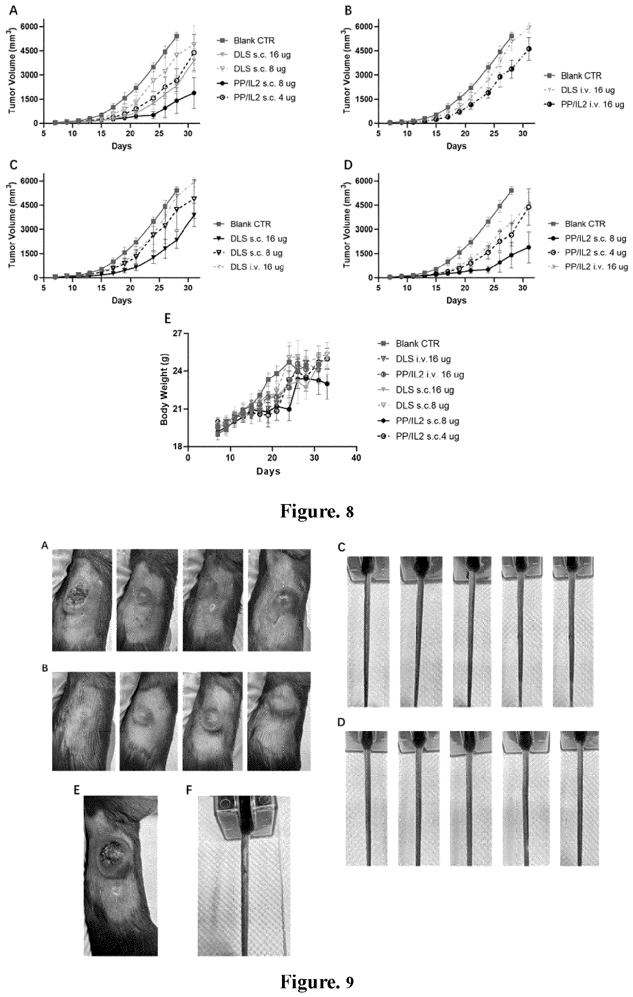

(1) B16F10 cells thawed from liquid nitrogen were cultured in DMEM medium (containing 10% fetal bovine serum) for 5-7 days, and passaged more than 3 times to ensure that the cells are in a normal state before use. On the day of the experiment, cells should grow to 80%-90% confluence and be in a good state. The cells were digested with 0.05% trypsin, neutralized with 10 times the volume of the fresh medium, centrifuged at 700×g to discard the supernatant, re-suspended in sterile PBS and counted. The cell suspension was adjusted to a density of 2×10⁶/ml with sterile PBS and placed on ice for later use.
(2) The female wild-type C57BL/6 mice aged 6-8 weeks were inoculated with the B16F10 cell suspension subcutaneously on the right back by using a 1 ml sterile syringe. Each mouse was injected with 0.1 ml of the cell suspension, that is, 2×10⁵ B16F10 cells (the cell suspension should be mixed thoroughly before each pipetting).
(3) On day 10 after inoculation, the mice had a visible tumor with a volume of 40±3 mm³ growing at the inoculation site. Mice with tumors of appropriate size and shape were grouped, so that the distribution and the mean value of the tumor volumes in each group were substantially the same. A total of 8 experimental groups were set up, with 5 mice in each group:
   A. Blank control group (Blank CTR)
   B. Positive reference DLS, tail vein administration group (DLS i.v. 16 µg)
   C. IL2/PP formulation, tail vein administration group (IL2/PP i.v. 16 µg)
   D. Positive reference DLS, high dose subcutaneous administration group (DLS s.c. 16 µg)
   E. Positive reference DLS, low dose subcutaneous administration group (DLS s.c. 8 µg)
   F. IL2/PP formulation, high dose subcutaneous administration group (IL2/PP s.c. 8 µg)
   G. IL2/PP formulation, low dose subcutaneous administration group (IL2/PP s.c. 4 µg)
(4) Starting from day 8 after inoculation, each mouse was injected with 0.1 ml of different formulations with corresponding concentrations (the actual dosage of IL-2 was 4, 8, and 16 µg and please refer to the above description of individual groups for the specific doses) once a day for 8 days for the subcutaneous administration group and for 4 days for the tail vein administration group according to the administration route and dosage of the group to which the mouse belonged (the blank control group did not receive any treatment).
(5) The IL2IPP formulation was prepared according to the method described in Example 1. The lyophilized powder of the positive reference DLS was reconstituted with water for injection to prepare the injection. Both were quantified by BCA and adjusted to the required concentration.
(6) The state of the mice was continuously observed from the completion of grouping. The tumor volume and body weight changes of the tumor-bearing mice were measured three times a week, the death was recorded and the toxic and side effects on the mice in each group were observed during the administration.
(7) The experimental results **(****Fig. 8 and Fig. 9****)** showed that, under the treatment of this administration regimen,
   ①when administered by subcutaneous injection, both the IL2/PP formulation and the DLS formulation significantly inhibited the growth of tumor in an obvious dose-dependent manner, that is, the higher the dose, the better the therapeutic effects, and compared with the DLS formulation, the IL2IPP formulation had a significantly better tumor inhibitory effect and achieved the same therapeutic effect at a much smaller dose and a better therapeutic effect at only 1/4 of the dose of the DLS formulation;
   ②when administered by tail vein injection, the IL2IPP formulation had a significantly better tumor inhibitory effect than the DLS formulation, which had an insignificant tumor inhibitory effect compared with blank control group;
   ③when treated with IL2/PP formulation, the subcutaneous administration had a significantly better effect than the tail vein administration and required only a smaller dose, and there was a significant difference between the two administration routes;
   ④ when treated with DLS formulation, the subcutaneous administration also had better effects than the tail vein administration;
   ⑤ the IL2/PP formulation slightly prolonged the survival of the tumor-bearing mice compared with the blank control group and the two IL2/PP subcutaneous administration groups each had a mouse in which tumor was completely eliminated, while any of the other groups did not;
   ⑥ when administered continuously by either subcutaneous injection or tail vein injection, the IL2/PP formulation was much safer than the DLS formulation and almost had no adverse effects, while the DLS formulations had very significant and serious side effects.

In summary, when administered either by subcutaneous injection or tail vein injection, the IL2IPP formulation was significantly better than the prior-art positive reference DLS in enhancing the anti-tumor effect and improving safety. Especially, when administered subcutaneously, the IL2/PP formulation was able to achieve the same therapeutic effect as the DLS formulation at a much smaller dose.

### Example 6. Validation of selective effect of IL2/PP formulation on cells with different receptors in the CT26-Balb/C mouse tumor-bearing model

(1) CT26 cells thawed from liquid nitrogen were cultured in RPMI-1640 medium (containing 10% fetal bovine serum) for 5-7 days, and passaged more than 3 times to ensure that the cells are in a normal state before use. On the day of the experiment, cells should grow to 80%-90% confluence and be in a good state. The cells were digested with 0.05% trypsin, neutralized with 10 times the volume of the fresh medium, centrifuged at 700×g to discard the supernatant, re-suspended in sterile PBS and counted. The cell suspension was adjusted to a density of 5×10⁶/ml with sterile PBS and placed on ice for later use.
(2) The female wild-type Balb/C mice aged 6-8 weeks were inoculated with the CT26 cell suspension subcutaneously on the right back by using a 1 ml sterile syringe. Each mouse was injected with 0.1 ml of the CT26 cell suspension, that is, 5×10⁵ CT26 cells.
(3) On day 8 after inoculation, the mice had a visible tumor with a volume of 80±15 mm³ growing at the inoculation site. Mice with tumors of appropriate size and shape were grouped, so that the distribution and the mean value of the volumes in each group were substantially the same. A total of 2 experimental groups were set up, with 3 mice in each group:
   A. Positive reference DLS, intraperitoneal administration group
   B. IL2/PP formulation, intraperitoneal administration group
(4) Starting from day 8 after inoculation, each mouse was injected intraperitoneally with 0.1 ml of the corresponding formulation (the actual dosage of IL-2 was 10 µg) once a day for 10 days according to the group to which the mouse belonged.
(5) The IL2IPP formulation was prepared according to the method described in Example 1. The lyophilized powder of the positive reference DLS was reconstituted with water for injection to prepare the injection. Both were quantified by BCA and adjusted to the required concentration.
(6) On day 19 after inoculation, the mice were euthanized by cervical dislocation and the spleen and tumor tissues were stripped from the mice to extract lymphocytes for detection.

For spleen, the sample was crushed and fully ground on a 70 µm filter mesh, the filtered suspension was centrifuged at 500×g for 5 minutes, the pellet was re-suspended with the ACK reagent to lyse erythrocytes, the obtained cell suspension was filtered with the filter mesh and centrifuged again, and the cells were re-suspended in the FACS buffer, counted and diluted to an appropriate concentration for fluorescent antibody staining.

For tumor tissue, the sample was cut into pieces, added with 2 mg/ml collagenase IV solution and digested in a constant temperature shaker at 37°C for 1-2 hours, the digested tissue sample was ground and filtered on a 70 µm filter mesh, the filtered suspension was centrifuged at 500×g for 5 minutes, the pellet was re-suspended with the ACK reagent to lyse erythrocytes, the obtained cell suspension was filtered with the filter mesh and centrifuged again, and the cells were re-suspended in the FACS buffer, counted, and diluted to an appropriate concentration for fluorescent antibody staining.

(7) Cell staining: 2×10⁶ cells were used for each sample, and the antibody staining protocols were as follows:
regulatory Tregs: anti-mouse CD45-BV605, anti-mouse CD4-BV421, and anti-Foxp3-PE;
CD8+ effector T cells: anti-mouse CD45-BV605, anti-mouse CD3-FITC, and anti-mouse CD8-PE-Cy7.
Foxp3 is an intracellular antigen, which requires fixation, permeabilization and staining posterior to staining of other surface antigens. After staining, the cells were washed and suspended with the FACS buffer, detected by a flow cytometer (BD LSR Fortessa), and analyzed by FlowJo X software.

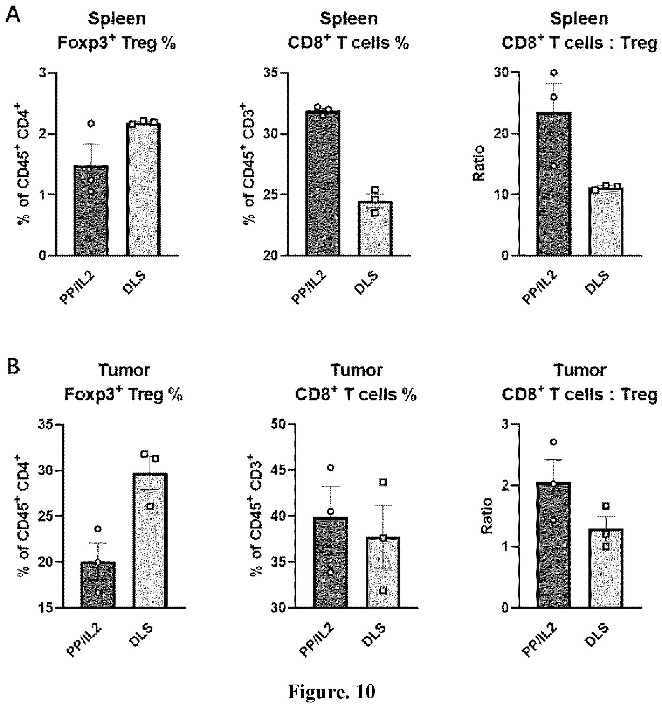

(8) The experimental results **(****Fig. 10****)** showed that, by this administration regimen,
①the IL2/PP formulation and the DLS formulation were significantly different in the ability of activating cell subsets with different receptors, and the IL2IPP formulation was capable of activating a higher percentage of CD8+ effector T cells while reducing the percentage of the activated immunosuppressive Tregs compared with the positive reference DLS formulation;
② the DLS formulation mainly activated the cell subset of immunosuppressive Tregs expressing high-affinity IL2 receptor, and showed a low capability of activating functional CD8+ effector T cells which express only medium-affinity IL2 receptors;
③the IL2/PP formulation showed significantly reduced activation of Tregs and significantly increased percentage of activated CD8+ effector T cells, and this trend was very obvious in spleen and tumor tissues;
④in the two different samples, namely the spleen and tumor tissues, although the percentages of Tregs and CD8+ T cells and the ratio of the two cells were different in value, the differences and trends caused by the two formulations were consistent, indicating that the selective effects of the two formulations on target cells were substantially the same in different immune environments.

In summary, **compared with the positive reference (the DLS formulation), the IL2IPP formulation had a significant selective effect on the activation of IL2 target cells, it no longer preferentially activated Treg subsets, but activated more CD8+ effector T cells.** This suggests that the IL2IPP formulation has changed the structure of the IL2 protein itself, and is more likely to directly activate effector T cells with tumor therapeutic effects. This is of great significance for improving various limitations of IL2 protein in clinical use.

### Example 7. Determination of effects of G-CSF/PP formulation on the infiltration of spleen- and tumor-derived immune cells

### 1. Cell culture and tumor inoculation method

Culture of the CT26 cell line for murine colon carcinoma model with RPMI-1640 medium (containing 10% fetal bovine serum). The CT26 tumor cells-containing cryopreservation tube was taken out of the liquid nitrogen storage tank, and put into a 37°C water bath for rapid thawing. The cell suspension was then transferred into a centrifuge tube containing 10 ml of the culture medium, and centrifuged at 350×g for 5 min to discard the supernatant. After re-suspended with fresh medium, the cells were transferred to a cell culture flask, and added with 10-15 ml of the medium to suspend the precipitated cells. After the cell concentration was adjusted, the cells were cultured in a 37°C incubator with a humidified atmosphere of 5% CO₂. During the culture, the cells were observed every day and replaced with the fresh medium in time.

Tumor cell inoculation: when the cells grew to 90% confluence, they were digested with 0.05% trypsin and sub-cultured at a ratio of 1:3. On the day of the experiment, the cells in a good growth state and growing to 90% confluence were digested with trypsin, neutralized with the fresh medium, centrifuged at 350 g to discard the supernatant, re-suspended in sterile PBS, and counted. The cell suspension was adjusted to a density of 5×10⁶/ml for later use. Each mouse was subcutaneously inoculated with 5×10⁵ cells.

### 2. Preparation of G-CSF/PP formulation

With reference to the preparation method of the G-CSF/PP formulation in Example 1, a stock solution with a concentration of 0.2 mg/mL was prepared for future use.

### 3. Experimental protocols: Detection of infiltration of MDSC, DC, CD8+ T cells and regulatory T cells (Treg) in the spleen and tumor tissues of tumor-bearing mice

(1) On day 0, female Balb/c mice were subcutaneously inoculated with CT26 tumor cells. On day 6, all mice developed tumors, which were begun to be monitored for growth daily and found to have an average tumor volume reaching 80-100 mm³ by day 11, and the tumor-bearing mice were randomly divided into groups (grouped as follows):
   a) Control group (tumor-bearing mice group, CTR group)
   b) Positive control group (protein bulk, used to prepare G-CSF formulation, purchased from Sihuanshengwu)
   c) G-CSF/PP group
   d) Normal mouse group (Normal)
(2) After grouping, the mice were injected subcutaneously with different formulations continuously for 5 days. The positive control was used in the G-CSF group, with a dosage of 20 µg/mouse/day. The prepared G-CSF/PP formulation was used in the G-CSF/PP group, with a same dosage of 20 µg/mouse/day.
(3) On day 17, the spleen and tumor were dissected from the tumor-bearing mice, weighed and recorded, the spleen was ground and filtered on a 70 µm sieve mesh to obtain a single cell suspension, and the tumor tissue was digested enzymatically, ground and filtered through a 70 µm sieve mesh to obtain a single cell suspension. The proportion and total number of spleen- and tumor-derived MDSCs (CD45⁺CD11b⁺Gr-1⁺), DCs (CD45⁺CD11c⁺MHCII⁺), total T cells (CD45⁺CD3⁺), CD4⁺ T cells (CD45⁺ CD3⁺CD4⁺) and Tregs (CD45⁺CD4⁺CD25⁺) were then detected by flow cytometry (Fig. 12).

### Result analysis:

In the spleen, G-CSF/PP had similar effects on the number of myeloid-derived cells and MDSC cells to the positive control, almost had no effect on the number of T cells (CD4⁺ and Treg), **and had a similar leukocyte elevation effect to the G-CSF protein bulk.** In the tumor, **G-CSF/PP significantly reduced the number of MDSCs and Tregs compared with the G-CSF protein bulk.** In summary, G-CSF/PP effectively reduced the immune-related side effects of the G-CSF protein.

Finally, it should be noted that the above examples are only used to help those skilled in the art to understand the essence of the present invention, and are not intended to limit the protection scope of the present invention.

## Claims

1. A cytokine protein pharmaceutical formulation, which is a water-soluble formulation, comprising
(1) a cytokine protein with a concentration of 40-20000 µg/ml, preferably, a cytokine protein with a concentration of 0.1-20 mg/ml, and more preferably, a cytokine protein with a concentration of 1-10 mg/ml;
(2) polyethylene glycol-distearoyl phosphatidylethanolamine (PEG-DSPE); and
(3) a necessary pH regulator/buffer;
the PEG-DSPE and the cytokine protein have a molar ratio of is 8-100:1;
preferably, the PEG-DSPE and the cytokine protein have a molar ratio of 10-80:1;
more preferably, the PEG-DSPE and the cytokine protein have a molar ratio of 15-50:1;
most preferably, the PEG-DSPE and the cytokine protein have a molar ratio of 20-40:1;
the cytokine protein is a human-derived cytokine with a structure comprising 3-8 α-helices and a molecular weight of 10-30 kDa.

2. The pharmaceutical formulation according to claim 1, wherein,
the cytokine protein is a human-derived wild-type cytokine protein with a structure only comprising α helix/helices,
preferably, the cytokine protein is a recombinantly expressed cytokine protein with a structure only comprising 4-6 α-helices and a molecular weight of 10-20 KDa;
more preferably, the cytokine is IL2, IL4, IL7, IL9, IL10, IL15, IL21, G-CSF, GM-CSF, IFN, EPO, GF or TNF.

3. The pharmaceutical formulation according to claim 1 or 2, wherein,
the PEG-DSPE has a purity of ≥95%, with a PEG chain having a dispersity of ≤1.1;
preferably, the PEG-DSPE molecule has a PEG chain having a length of 1000-5000 Da;
more preferably, the PEG-DSPE molecule has a PEG chain having a length of 1500-2500 Da;
most preferably, the PEG-DSPE molecule has a PEG chain having a length of 2000 Da (PEG2000-DSPE).

4. The pharmaceutical formulation according to any one of claims 1-3, wherein,
the cytokine protein pharmaceutical formulation is adjusted with the pH regulator to have a pH of 5.5-6.0;
the pH regulator/buffer includes, but is not limited to, hydrochloric acid, acetic acid, sodium/potassium hydroxide, sodium bicarbonate, sodium dihydrogen carbonate, sodium hydrogen phosphate, sodium dihydrogen phosphate, and amino acid pH regulators/buffers such as glycine or histidine.

5. The pharmaceutical formulation according to any one of claims 1-4, wherein,
the cytokine protein pharmaceutical formulation is further lyophilized to prepare a lyophilized formulation;
the lyophilized formulation further comprises a lyoprotectant, including but not limited to glycerin, glycine, sucrose, lactose, albumin, histidine, mannitol or a combination thereof.

6. A method of preparing the cytokine protein pharmaceutical formulation according to any one of claims 1-5, comprising
(1) preparing an aqueous solution with a pH of 3.0-5.5, preferably by diluting glacial acetic acid to a concentration of 10 mM with deionized water and adjusting the pH to 3.0-3.5;
(2) weighing an amount of cytokine protein dry powder, adding the aqueous solution with low pH to the cytokine protein dry powder, and mixing thoroughly to obtain a cytokine protein suspension;
(3) weighing a corresponding amount of PEG-DSPE dry powder according to the amount of the cytokine protein in step (2), adding the PEG-DSPE dry powder to the cytokine protein suspension, and mixing thoroughly;
(4) heating in a water bath at 40°C-60°C for 10-30 minutes; and
(5) allowing the solution obtained in step (4) to stand to balance to room temperature, adjusting its pH to 5.5-6.0, and allowing the solution to stand for another 10-60 minutes to obtain the cytokine protein pharmaceutical formulation;
the cytokine protein pharmaceutical formulation prepared by the method has a protein recovery rate of ≥90%, and the recovery rate is a ratio of the protein content in the cytokine protein pharmaceutical formulation to the added protein amount.

7. The method according to claim 6, wherein,
the PEG-DSPE has a purity of ≥95%, with a PEG chain having a dispersity of ≤1.1;
preferably, the PEG-DSPE molecule has a PEG chain having a length of 1000-5000 Da;
more preferably, the PEG-DSPE molecule has a PEG chain having a length of 1500-2500 Da;
most preferably, the PEG-DSPE molecule has a PEG chain having a length of 2000 Da (PEG2000-DSPE).

8. The method according to claim 6 or 7, wherein the method further comprises,
(6) diluting the cytokine protein formulation in the solution obtained in step (5) to an appropriate concentration with the same buffer, or concentrating the cytokine protein formulation in the solution obtained in step (5) by ultrafiltration to an appropriate concentration;
(7) sterilizing the cytokine protein formulation by filtration.

9. Use of the cytokine protein pharmaceutical formulation according to any one of claims 1-5 in the manufacture of a medicament; preferably, the medicament is administered by subcutaneous injection.

10. Use of polyethylene glycol-distearoyl phosphatidylethanolamine (PEG-DSPE) in the manufacture of a formulation for changing affinity between a protein of interest and its receptor, the protein of interest comprises at least one α-helix; preferably, the protein of interest comprises four to six α-helices; more preferably, the protein of interest is IL2, IL4, IL7, IL9, IL15, IL21, G-CSF or GM-CSF;
Said changing affinity between the protein of interest and its receptor is reducing binding ability of the protein to a low-affinity receptor; preferably, the low-affinity receptor has an affinity (Kd value) of ≥ 10⁻⁶ M to the protein; more preferably, the low-affinity receptor has an affinity (Kd value) of ≥ 10⁻⁷ M to the protein; and most preferably, the low-affinity receptor has an affinity (Kd value) of ≥ 10⁻⁸ M to the protein.

11. The use according to claim 10, wherein in the formulation,
(1) the PEG-DSPE molecule and the α-helix of the protein of interest has a ratio of 2-25: 1 in number; preferably, the PEG-DSPE molecule and the α-helix of the protein of interest has a ratio of 3-20: 1 in number; more preferably, the PEG-DSPE molecule and the α-helix of the protein of interest has a ratio of 4-15 in number: 1; or
(2) the PEG-DSPE and the protein of interest has a molar ratio of 8-100: 1; preferably, the PEG-DSPE and the protein of interest has a molar ratio of 10-80: 1; more preferably, the PEG-DSPE and the protein of interest has a molar ratio of 15-50: 1; most preferably, the PEG-DSPE and the protein of interest has a molar ratio of 20-30: 1.

12. The use according to claim 10 or 11, wherein,
the PEG-DSPE has a purity of ≥95%, with a PEG chain having a dispersity of ≤1.1;
preferably, the PEG-DSPE molecule has a PEG chain having a length of 1000-5000 Da;
more preferably, the PEG-DSPE molecule has a PEG chain having a length of 1500-2500 Da;
most preferably, the PEG-DSPE molecule has a PEG chain having a length of 2000 Da (PEG2000-DSPE).

13. An IL2IPP formulation, which is a water-soluble formulation, comprising:
(1) IL2 protein with a concentration greater than 1 mg/ml, preferably with a concentration of 1-10 mg/ml;
(2) polyethylene glycol-distearoyl phosphatidylethanolamine (PEG-DSPE); preferably, the PEG-DSPE molecule has a PEG chain having a length of 2000 Da (PEG2000-DSPE), and the PEG-DSPE has a purity of ≥95%, with a PEG chain having a dispersity of ≤1.1; and
(3) a necessary pH regulator/buffer;
the PEG-DSPE and the IL2 protein has a molar ratio of 8-100: 1;
preferably, the PEG-DSPE and the IL2 protein has a molar ratio of 10-80: 1;
more preferably, the PEG-DSPE and the IL2 protein has a molar ratio of 15-50: 1;
most preferably, the PEG-DSPE and the IL2 protein has a molar ratio of 20-30:1;
the IL2 protein is expressed in *Escherichia coli* and purified, and has an amino acid sequence as shown in SEQ ID NO.1.

14. The formulation according to claim 13, wherein the IL2IPP formulation is adjusted to have a pH of 5.5-6.0 by using the pH regulator.

15. A method of preparing the IL2/PP formulation according to claim 13 or 14, comprising
(1) preparing an aqueous solution with a pH of 3.0-5.5, preferably by diluting glacial acetic acid to a concentration of 10 mM with deionized water and adjusting the pH to 3.0-3.5;
(2) weighing an amount of IL2 protein dry powder, adding the aqueous solution with the pH of 3.0-3.5 to the IL2 protein dry powder, and mixing thoroughly to obtain an IL2 suspension;
(3) weighing a corresponding amount of PEG-DSPE dry powder according to the amount of the IL2 protein in step (2), adding the PEG-DSPE dry powder to the IL2 suspension, and mixing thoroughly;
(4) heating in a water bath at 40°C-60°C for 10-30 minutes; and
(5) allowing the solution obtained in step (4) to stand to balance to room temperature, adjusting its pH to 5.5-6.0, and allowing the solution to stand for another 10-60 minutes to obtain the IL2IPP formulation;
preferably, the PEG-DSPE molecule has a PEG chain having a length of 2000 Da (PEG2000-DSPE), and the PEG-DSPE has a length of ≥95%, with a PEG chain having a dispersity of ≤1.1;
the IL2/PP formulation prepared by the method has a IL2 protein recovery rate of ≥90%.

16. Use of the IL2/PP formulation according to claim 13 or 14
(1) in the manufacture of an antitumor medicament;
(2) in the manufacture of a formulation for activating cellular immune response;
(3) in the manufacture of an anti-tumor combined formulation; preferably, the combined formulation further comprises an antibody or an antibody construct, a cytotoxic agent, an immunotherapeutic or cellular therapeutic agent;
the medicament or formulation has lower toxicity and/or side effects compared to the IL2 formulation in the form of a DLS formulation;
said activating cellular immune response refers to, *in vivo* or *in vitro,*
(1) up-regulating proliferation of CD8+ T cells, and/or
(2) inducing differentiation of T cells into CD8+ T cells, and/or
(3) inhibiting differentiation of T cells into Tregs; and/or
(4) increasing ratio of CD8+T cells/Tregs;
preferably, said activating cellular immune response means that
the IL2/PP formulation, compared to the IL2 formulation in the form of a DLS formulation (positive reference formulation) or the formulation in the form of a free-protein solution, is capable of, *in vivo* or *in vitro:*
(1) up-regulating proliferation of CD8+ T cells more, and/or
(2) inducing differentiation of T cells into CD8+ T cells more, and/or
(3) inhibiting differentiation of T cells into Tregs more;
(4) increasing ratio of CD8+T cells/Tregs;
preferably, the medicament or formulation is administered by subcutaneous injection.

17. A G-CSF/PP formulation, which is a water-soluble formulation, comprising
(1) G-CSF protein with a concentration greater than 1 mg/ml, preferably with a concentration of 1-10 mg/ml;
(2) polyethylene glycol-distearoyl phosphatidylethanolamine (PEG-DSPE); preferably, the PEG-DSPE molecule has a PEG chain having a length of 2000 Da (PEG2000-DSPE), and the PEG-DSPE has a purity of ≥95%, with a PEG chain having a dispersity of ≤1.1; and
(3) a necessary pH regulator/buffer;
the G-CSF protein is expressed in *Escherichia coli* and purified, and has an amino acid sequence as shown in SEQ ID NO.2.

18. The formulation according to claim 17, wherein the G-CSF/PP formulation is adjusted to have a pH of 5.5-6.0 by using the pH regulator.

19. A method of preparing the G-CSF/PP formulation according to claim 17 or 18, comprising
(1) preparing an aqueous solution with a pH of 3.0-5.5, preferably by diluting glacial acetic acid to a concentration of 10 mM with deionized water and adjusting the pH to 3.0-3.5;
(2) weighing an amount of G-CSF protein dry powder, adding the aqueous solution with the pH of 3.0-3.5 to the G-CSF protein dry powder, and mixing thoroughly to obtain a G-CSF suspension;
(3) weighing a corresponding amount of PEG-DSPE dry powder according to the amount of G-CSF protein in step (2), adding the PEG-DSPE dry powder to the G-CSF suspension, and mixing thoroughly;
(4) heating in a water bath at 40°C-60°C for 10-30 minutes; and
(5) allowing the solution obtained in step (4) to stand to balance to room temperature, adjusting its pH to 5.5-6.0, and allowing the solution to stand for another 10-60 minutes to obtain the G-CSF/PP formulation;
preferably, the PEG-DSPE molecule has a PEG chain having a length of 2000 Da (PEG2000-DSPE), and the PEG-DSPE has a purity of ≥95%, with a PEG chain having a dispersity of ≤1.1;
the G-CSF/PP formulation prepared by the method has a G-CSF protein recovery rate of >90%.

20. Use of the formulation according to claim 17 or 18 in the manufacture of a medicament for treating febrile neutropenia (FN); preferably, the febrile neutropenia is caused by radiotherapy or chemotherapy of tumors or organ transplantation; preferably, the medicament is administered by subcutaneous injection.
